# EUROPEAN PATENT APPLICATION

(11) **EP 4 450 612 A1**
(43) Date of publication of application: **23.10.2024**
(21) Application number: 22907504.9
(22) Date of filing: 15.12.2022
(51) Int. Cl.: C12N 5/071, C12N 5/0775, C07K 14/78

(54) **NOVEL NASAL MUCOSAL MEMBRANE CELL SHEET**

(30) Priority: 15.12.2021 JP 2021203126
(71) Applicant: The Jikei University, Tokyo 105-8461 (JP)
(72) Inventor: KOJIMA Hiromi, Tokyo 105-8461 (JP); YAMAMOTO Kazuhisa, Tokyo 105-8461 (JP); MORINO Tsunetaro, Tokyo 105-8461 (JP); KASAI Yoshiyuki, Tokyo 105-8461 (JP)
(74) Representative: Jones, Nicholas Andrew
(86) International application number: PCT/JP2022/046169
(87) International publication number: WO 2023/112981

(57) **Abstract**

An object of the present invention is to provide a cultured cell sheet that can solve problems accompanied by defects of mucosal tissue as described above, such as inflammation in mucosal tissues covering bone tissues and granulation tissue formation. In order to solve the problems described above, the present inventors have conducted research and development with examinations from various angles. As a result, it has been found that a cultured cell sheet obtained by culturing cells collected from a nasal mucosal tissue as a source under specific conditions, and reconstructing the cells into a sheet are engrafted onto mucosal tissue defective sites, such as due to inflammation on the surface of middle ear bone tissues and granulation tissue formation; promote the regeneration of mucosal tissues, such as those covering bone tissues; efficiently reduce inflammation that occurs in a middle ear bone tissue; and prevent fibrosis, granulation tissue formation, defective epithelial formation, and the like that are characteristic of bone tissue inflammation, thereby demonstrating that the cultured cell sheet can solve the above-described problems. It is also found that a cultured cell sheet made from cells collected from a nasal mucosal tissue of the present invention is also useful for preventing inflammation of bone tissues in other areas of the body other than the middle ear.

## Description

### TECHNICAL FIELD

The present invention relates to cultured cell sheets made from cells collected from a nasal mucosal tissue useful in the field of medicine, production methods, and methods of their use.

### BACKGROUND ART

Humans perceive aerial vibrations as sounds, having frequencies of about 20-20,000 Hz that reach the ear auricles. Sounds enter the external auditory canal as air vibrations, which are in turn transmitted from the tympanic membrane to the three ear ossicles in the middle ear cavity, where the vibrations are amplified and transmitted to the cochlea in the inner ear. The vibrations become a jolt of the lymph filling the inner ear, and this stimulation is transmitted from the hair cells present in the cochlea to the cochlear nerve. Signals transmitted to the cochlear nerve pass through the brainstem and diencephalon to the auditory area of the cerebral cortex, where they are perceived as sounds. Impairment of any of the conduction pathways causes hearing loss. The middle ear cavity is covered by the middle ear mucosa, which also serves as a periosteum, and remains well aerated due to the gas ventilation capacity of the mucosa. This aeration allows sounds to be efficiently transmitted to the inner ear. Although the functions of the mucosa play an important role in the middle ear, no method has been established to regenerate the damaged middle ear mucosa.

Adhesive otitis media and middle ear cholesteatoma are known as representative intractable middle ear diseases that cause hearing loss. Adhesive otitis media is a disease in which the tympanic membrane fails to maintain its original and normal position and adheres to the bone wall in the middle ear cavity, resulting in loss of air in the middle ear cavity. Adhering tympanic membrane is unable to transmit sounds properly due to prevention of vibration, resulting in hearing loss. In addition, adhering tympanic membrane is prone to infection and repeated otorrhea, and in advanced stages, can lead to middle ear cholesteatoma. Middle ear cholesteatoma is a disease where keratinized stratified squamous epithelium, which is continuous from the external auditory canal skin, containing keratinization and desquamation products, invades the middle ear and destroying the surrounding bone tissues, thereby causing various complications. The destruction of the ear ossicles leads to hearing loss, and if the inflammation or bone destruction spreads to the inner ear, it causes irreversible cochlear hearing loss. Furthermore, the anatomical characteristics of the middle ear cause various other serious complications, such as dizziness, facial paralysis, and intracranial complications such as meningitis and brain abscess.

The fundamental treatment for adhesive otitis media and middle ear cholesteatoma is a middle ear surgery called tympanoplasty. One of the goals of this treatment is to improve hearing, which requires the presence of air in the middle ear cavity and transmission of tympanic membrane vibrations to the inner ear via the ear ossicles without loss. To form an ideal middle ear cavity after surgery, it is important to regenerate the middle ear mucosa, thereby restoring physiological ability of gas ventilation and preventing adhesion of the tympanic membrane. However, because the functions of the middle ear mucosa are basically impaired in case of adhesive otitis media or middle ear cholesteatoma, postoperative regeneration of the middle ear mucosal epithelium is often delayed, making it difficult to create an effective pneumatic space. Especially in cases of adhesive otitis media where the tympanic membrane is depressed and adheres to the inner wall of the middle ear cavity, it is difficult to preserve the middle ear mucosa because the bone surface of the middle ear is exposed when the lesion is removed during surgery. For this reason, postoperative hearing improvement in patients with adhesive otitis media is much poorer than in patients with other middle ear diseases.

On the other hand, techniques for middle ear cholesteatoma include canal wall up tympanoplasty that preserves the posterior wall of the external auditory canal and canal wall down tympanoplasty that remove the posterior wall. The canal wall up tympanoplasty is superior in that it preserves the physiological morphology of the external auditory canal, but the disadvantage of this technique is the high incidence of postoperative recurrence. Although persistent relapse caused by lesions left after the surgery can be prevented, for example, with the use of endoscopes in recent years, it is difficult to ensure prevention of recurrent recurrence caused by readhesion or redepression after the surgery. On the other hand, the canal wall down tympanoplasty can prevent recurrent recurrence, but impair the physiological morphology of the external auditory canal, possibly resulting in postoperative mastoid cavity disorders, called postoperative cavity problems. The ideal technique is to preserve the posterior wall of the external auditory canal and allow for formation of the middle ear cavity with good postoperative pneumatization by canal wall up tympanoplasty. However, there is no definitive surgical technique, and there are limitations with conventional tympanoplasty alone, so that currently no better treatment results can be expected than have been achieved so far.

Accordingly, if it becomes possible to regenerate in an early stage after surgery the middle ear mucosa impaired, it will be possible to prevent readhesion of the tympanic membrane for adhesive otitis media, and also to prevent recurrent recurrence while preserving the posterior wall of the external auditory canal for middle ear cholesteatoma. This is thought to be a significant improvement in the prevention of recurrence, which has been difficult to achieve in the past. Until now, the major issue has been how to regenerate the middle ear mucosa in an early stage. In hopes of preventing postoperative redepression and readhesion of the tympanic membrane and regenerating the middle ear mucosa, various studies have attempted direct transplantation of various types of mucosa (Non-Patent Document 1), but there is still no established therapy.

Under such background, the present inventors have been promoted human clinical application of a novel therapy using cell sheet engineering to regenerate the middle ear mucosa for refractory middle ear diseases. The inventors have previously studied regeneration of the middle ear mucosa. The inventors have prepared an artificial middle ear mucosa in three dimensions (Non-Patent Document 2), and have transplanted it to a rabbit model of impaired middle ear mucosa to find that good postoperative regeneration of the mucosa was observed (Non-Patent Document 3). Subsequently, toward the clinical realization of regenerative medicine of the middle ear mucosa in humans, the inventors have been studied the regeneration of middle ear mucosa using somatic stem cells (Non-Patent Document 5) by using cell culture substrates coated with a temperature-responsive polymer based on cell sheet engineering (Patent Document 1, Non-Patent Document 4). Regenerative medicine using cell sheet engineering has already been successfully applied to human clinical practice, such as in the cornea and esophagus, which has provided good therapeutic effects. We focused on the nasal mucosa, which can be collected minimally invasively in an outpatient setting, as a cell source for cell sheet production and is anatomically contiguous and histologically similar to the middle ear mucosa. In order to promote regeneration of the middle ear mucosa after surgery and prevent readhesion of the tympanic membrane, we have developed a new therapy in which an autologous cultured epithelial cell sheet is prepared using nasal mucosa with a culture substrate coated with a temperature-responsive polymer, and is transplanted to the postoperative middle ear cavity. Preclinical animal experiments in rabbits using cell inserts for cell culture coated with a temperature-responsive polymer have been successful (Non-Patent Document 6), and human nasal mucosal epithelial cell sheets have also been successfully produced (Patent Document 2).

It is known that loss of mucosal tissues on the surface of the middle ear bone causes problems such as inflammation of the bone tissue and granulation tissue formation. Once such problems occur, effective treatment is by means of removal of the granulation tissues or suppression of inflammation of the surface of the bone tissue, but there have been problems such as recurrence. Even use of the conventional human nasal mucosal epithelial cell sheets developed by the inventors (Patent Document 2) has failed to solve the problems.

### PRIOR ART DOCUMENTS

### PATENT DOCUMENTS

[Patent Document 1] Japanese Patent Application Laid-Open (JP-A) No. H02-211865
[Patent Document 2] Japanese Patent No. 6021643

### NON-PATENT DOCUMENTS

[Non-Patent Document 1] Otol Jpn, 5: 574-577. (1995)
[Non-Patent Document 2] Acta Oto-Laryngologica, 126: 801-810. (2006)
[Non-Patent Document 3] J Tissue Eng Regen Med, 10: E188-94. (2016)
[Non-Patent Document 4] J Tissue Eng Regen Med, 11: 1089-1096. (2017)
[Non-Patent Document 5] NPJ Regen Med, Article number: 6. (2017)
[Non-Patent Document 6] Biomaterials, 42: 87-93. (2015)

### SUMMARY OF THE INVENTION

### PROBLEMS TO BE SOLVED BY THE INVENTION

An object of the present invention is to provide a cultured cell sheet that can solve problems accompanied by loss of mucosal tissues as described above, such as inflammation on the surface of bone tissues and granulation tissue formation.

### MEANS FOR SOLVING THE PROBLEMS

In order to solve the problems described above, the present inventors have conducted research and development with examinations from various angles. As a result, it has been found that a cultured cell sheet obtained by culturing cells collected from a nasal mucosal tissue as a source under specific conditions, and reconstructing the cells into a sheet are engrafted onto mucosal tissue defective sites, such as due to inflammation on the surface of middle ear bone tissues and granulation tissue formation; promote the regeneration of mucosal tissues covering bone tissues and the like; efficiently reduce inflammation that occurs in a middle ear bone tissue; and prevent fibrosis, granulation tissue formation, defective epithelial formation, and the like that are characteristic of bone tissue inflammation, thereby demonstrating that the cultured cell sheet can solve the above-described problems. It is also found that a cultured cell sheet made from cells collected from a nasal mucosal tissue of the present invention is also useful for preventing inflammation of bone tissues in other areas of the body other than the middle ear.

More specifically, the present application provides the following aspects to solve the problems described above:
[1]: A cultured cell sheet, wherein the cultured cell sheet is made from cells collected from a nasal mucosal tissue as a source, and comprises 50 to 90% of undifferentiated cells relative to the total number of cells;
[2]: The cultured cell sheet according to [1], wherein the undifferentiated cells are positive for p63;
[3]: The cultured cell sheet according to [1] or [2], wherein the undifferentiated cells are nasal mucosal epithelial stem cells or nasal mucosal epithelial progenitor cells;
[4]: The cultured cell sheet according to [1] or [2], wherein the undifferentiated cells do not have cilia;
[5]: The cultured cell sheet according to [1] or [2], wherein the cell sheet is a mixture comprising nasal mucosal epithelial cells and nasal mucosal epithelial stem cells or nasal mucosal epithelial progenitor cells, along with containing any one or two or more cells of other epithelial cell or other epithelial stem cell, a mesenchymal stem cell, a fibroblast, a vascular endothelial cell, an endothelial progenitor cell, and an adipose-derived stem cell;
[6]: The cultured cell sheet according to [5], wherein the percentage of the fibroblasts contained in the cell sheet is 0 to 12%;
[7]: The cultured cell sheet according to [1] or [2], wherein the nasal mucosal tissue from which the cells of the source are derived is derived from an inferior turbinate mucosa, a middle turbinate mucosa, a superior turbinate mucosa, a nasal mucosa, or a paranasal sinus tissue;
[8]: The cultured cell sheet according to [1] or [2], wherein the nasal mucosal tissue is derived from either human, rabbit, dog, cat, pig, monkey, chimpanzee, rat, or mouse;
[9]: The cultured cell sheet according to [1] or [2], intended for the treatment of chronic otitis media, adhesive otitis media, middle ear cholesteatoma, chronic sinusitis, paranasal sinus neoplasm, external auditory canal tumor, external auditory canal atresia, chronic tonsillitis, pharyngeal cancer, laryngeal cancer, oral cancer, tongue cancer, endotracheal granulation, endotracheal cicatrization, tracheal stenosis, tracheal injury, esophageal cancer, vocal cord lesion, inflammatory bowel disease, anal ulcer, anal fissure, hemorrhoid, or anal fistula;
[10]: A method of producing a cultured cell sheet, wherein the cultured cell sheet is made from cells collected from a nasal mucosal tissue as a source and comprises 50-90% of undifferentiated cells relative to the total number of cells, and the method comprises the following steps of:
   (1) preparing mucosal epithelial cells as the source from the nasal mucosal tissue;
   (2) culturing the prepared mucosal epithelial cells to increase the number of the mucosal epithelial cells as the source;
   (3) seeding the obtained cells onto a cell culture substrate and culturing the cells to prepare a cultured cell sheet in which the cells cover the culture substrate surface on the culture substrate; and
   (4) detaching and collecting the cultured cell sheet prepared on the culture substrate while maintaining the sheet shape;
[11]: The method according to [10], wherein the mucosal epithelial cells as the source are prepared by a following step in the step (1):
   preparing the mucosal epithelial cells as the source by obtaining cells by an explant culture method in which the nasal mucosal tissue collected from a subject is left to stand directly on a cell culture substrate without enzyme treatment, and cultured in serum-containing medium or serum-free medium to allow the cells in the tissue to migrate into the medium;
[12]: The method of producing the cultured cell sheet according to [10] or [11], wherein the cell culture substrate used in the steps (1) and (2) is a dish type culture substrate, a flask type culture substrate, or a multiwell type culture substrate;
[13]: The method according to [10] or [11], wherein the mucosal epithelial cells as the source are prepared by following steps in the steps (3) and (4):
   (3) culturing the prepared mucosal epithelial cells on a cell culture substrate coated on its surface with a temperature-responsive polymer having varying hydration force in the temperature range of 0 to 80°C, in a temperature range in which the temperature-responsive polymer has weak hydration force to prepare a cultured cell sheet on the cell culture substrate; and
   (4) changing the temperature of the culture medium into a temperature at which the temperature-responsive polymer has strong hydration force to detach the cultured cell sheet from the cell culture substrate and obtain the cultured cell sheet while maintaining the sheet shape;
[14]: The method of producing the cultured cell sheet according to [10] or [13], wherein the cell culture substrate used in the step (3) is coated on the surface with one or a mixture of two or more of type I collagen, laminin, fibronectin, and Matrigel;
[15]: The method of producing the cultured cell sheet according to [10] or [13], wherein the temperature-responsive polymer used in the steps (3) and (4) is poly-(N-isopropylacrylamide);
[16]: The method of producing the cultured cell sheet according to [10] or [13], wherein the detaching process in the step (4) is adhering a carrier closely to the cultured cell and detaching the cultured cell together with the carrier;
[17]: The method of producing the cultured cell sheet according to [10] or [13], further comprising laminating the cell sheet detached in the step (4) on another cell sheet detached in the step (4), or repeating the operation to laminate cell sheets;
[18]: The method of producing the cultured cell sheet according to [10] or [13], wherein the number of the mucosal epithelial cells seeded at the start of the culturing in the step (3) is 3 × 10⁴ to 2 × 10⁵ cells/cm²;
[19]: The method of producing the cultured cell sheet according to [10] or [13], wherein feeder cells are used in the culturing in the step(s) (2) and/or (3); and
[20]: The method of producing the cultured cell sheet according to [10] or [13], wherein a serum-free medium is used in the culturing in the step (3).

### EFFECT OF THE INVENTION

The cultured cell sheet made from cells collected from a nasal mucosal tissue disclosed in the present invention can be efficiently engrafted onto the surface of middle ear bones, promote the regeneration of mucosal tissues covering bone tissues, and prevent fibrosis, granulation tissue formation, defective epithelial formation, and the like in the middle ear cavity. The cultured cell sheet made from cells collected from a nasal mucosal tissue is also used as a useful mucosa in cases of loss of mucosa in the nasal cavity and oral cavity. Furthermore, a cultured cell sheet made from cells collected from a nasal mucosal tissue of the present invention is also useful for preventing inflammation of bone tissues in other areas of the body other than the middle ear.

### BRIEF DESCRIPTION OF THE DRAWINGS

FIG. 1 shows that fibroblast-like cells and undifferentiated cells migrated from a nasal mucosal tissue in a serum-containing explant culture method.
FIG. 2 shows a cell sheet obtained by using feeder cells and preparing sheet cells on a cell culture substrate coated with a temperature-responsive polymer.
FIG. 3 shows specific observation for cell count in a tissue section after immunohistochemical staining for the expression of p63.
FIG. 4 shows a cell sheet collected from a cell culture substrate coated with a temperature-responsive polymer (left), and a cell sheet obtained from a cell insert coated with a temperature-responsive polymer (right), both of which do not use feeder cells and detached from the cell culture substrate.
FIG. 5 shows that fibroblast-like cells and undifferentiated cells migrated from a nasal mucosal tissue in a serum-free explant culture method.
FIG. 6 shows a cell sheet obtained without using feeder cells and by preparing sheet cells on a cell culture substrate coated with a temperature-responsive polymer.
FIG. 7 shows a cell sheet collected from a cell culture substrate coated with a temperature-responsive polymer, which does not use feeder cells and detached from the cell culture substrate.
FIG. 8 shows observation in a tissue section after immunohistochemical staining for the expression of p63 and PDGFRα.
FIG. 9 shows working processes where a nasal mucosa-like cell sheet of the present invention is transplanted to a rabbit whose mucosa in the middle ear cavity is previously removed, and then the effect of the bone tissue-regenerating cell sheet of the present invention is evaluated.
FIG. 10 shows a schematic flow where a cultured cell sheet made from cells collected from a nasal mucosal tissue according to the method described in Example 1 without use of feeder cells is transplanted to a human patient.
FIG. 11 shows the postoperative course of a patient after transplantation of a nasal mucosal epithelial cell sheet of the present invention.

### MODE FOR CARRYING OUT THE INVENTION

The present invention provides a cultured cell sheet made from cells collected from a nasal mucosal tissue. The present invention also provides a method comprising collecting cells from a nasal mucosal tissue under specific culture conditions, and finally preparing a sheet-shaped cultured cell sheet on a cell culture substrate. The present invention further provides a method of using the obtained cultured cell sheet.

### <Cell sheet>

According to the present invention, a cultured cell sheet made from cells collected from a nasal mucosal tissue, the cultured cell sheet containing 50 to 90% of undifferentiated cells relative to the total number of cells can be provided. Examination by the present inventors has demonstrated that undifferentiated cells are more likely to proliferate when cultured, have good adhesion to the transplantation site, and function to regenerate epithelial tissues at the transplantation site after transplantation. At the beginning of development, the present inventors have considered that nasal mucosal epithelial cells that have abundant cilia and are fully differentiated, i.e. not undifferentiated, are more useful. They have focused on cells with cilia, proposing that, for example, "less than 5% of cells with cilia result in disability of the transplanted cell sheet to fully show the effect of the present invention, and thus are undesirable" (Patent Document 2). They have found that, in subsequent examination, that undifferentiated cells (cells without cilia) show more desirable effect as a cell sheet, and that inclusion of 50-90% of undifferentiated cells relative to the total number of cells in a cell sheet results in increased engraftment rate of the cell sheet to a transplantation site, allowing for regeneration of epithelial tissues on the surface of the middle ear bone, thereby achieving the present invention.

In the present invention, undifferentiated cells constituting the cultured cell sheet made from cells collected from a nasal mucosal tissue are comprised in an amount of 50% or more, preferably 55% or more, more preferably 60% or more, and still more preferably 65% or more relative to the total number of cells in the cell sheet.

The undifferentiated cells can proliferate not only during culture but also at the transplantation site after transplantation, and serve to regenerate epithelial tissues at the transplantation site. Thus, in the case of less than 15% of undifferentiated cells relative to the total number of cells in the cell sheet, the cell sheet transplanted to the transplantation site cannot fully show the effect of the present invention. Undifferentiated cells according to the present invention refer to cells that are not pluripotent like iPS cells or ES cells but retain multipotency that allows them to differentiate into epithelial cells, ciliated cells, goblet cells, or the like.

Undifferentiated cells according to the present invention are positive for p63 as a marker characteristic. p63 is known to be a transcription factor that is essential for epithelial morphogenesis and sternness. In the present invention, p63 is a marker indicating cells having high sternness (i.e., undifferentiated cells) among cells collected from a nasal mucosal tissue. Whether the cells comprised in the cultured cell sheet are positive for p63 can be determined by detecting cells using an antibody against p63. Specifically, detection can be performed, for example, by a method in which a tissue section is prepared from a cultured cell sheet and immunostained to detect p63-positive cells; a method in which cells in a cultured cell sheet are enzymatically separated into discrete cells, and cells binding to an antibody against p63 are detected as p63-positive cells by flow cytometry; a method in which cells in a cultured cell sheet are enzymatically separated into discrete cells, and then p63 protein or p63 gene is detected by Western blotting, real-time PCR, or the like. Among the methods described above, either an immunostaining or flow cytometry method is preferably used in order to determine how many p63-positive cells (undifferentiated cells) exist in a plurality of cells.

The undifferentiated cells according to the present invention are preferably epithelial stem cells, and examples thereof include, but are not limited to, nasal mucosal epithelial stem cells, nasal mucosal epithelial progenitor cells, and mesenchymal stem cells. Cells comprised in the cultured cell sheet can be defined as epithelial stem cells based on the detection of the expression of p63 described above, the detection of the expression of cytokeratin 5, or the detection of the expression of cytokeratin 14. Undifferentiated cells comprised in the cultured cell sheet of the present invention are characterized, for example, as having no cilia and comprising no goblet cell as a form of cells that can be observed under a microscope.

As described above, the cultured cell sheet according to the present invention preferably comprises many undifferentiated cells. The cell sheet according to the present invention may comprise those other than epithelial stem cells, the type of which is not restricted at all. Examples include any one, or a mixture of any two or more epithelial cells other than nasal mucosal epithelial cell, a fibroblast, a vascular endothelial cell, an endothelial progenitor cell, a bone marrow-derived cell, an adipose-derived stem cell, and a mesenchymal stem cell. The respective content ratio of those cells contained is not also particularly limited.

Cells that may be comprised in the cultured cell sheet of the present invention can include a fibroblast, but the percentage is very low and is required to be in the range from 0% to 12%, preferably from 0% to 5%. In general, fibroblasts in a cultured cell sheet act to strengthen the structure of the cell sheet, and their inclusion in the range from 0.1% to 1% is considered resulting in increased strength of the cell sheet.

In accordance with the present invention, the content ratio of nasal mucosal cells having cilia (i.e., one of differentiated cells) relative to the total number of cells in the final cultured cell sheet made from cells collected from a nasal mucosal tissue is required to be less than 5%, preferably less than 4%, more preferably less than 3%, and most preferably less than 1%. Cultured cell sheets containing nasal mucosal cells with cilia in a content of 5% or more are undesirable because they show inferior engraftment properties onto the surface of the middle ear bone when transplanted to affected areas, and thus cannot sufficiently show the effect of the present invention.

Cells to be used as a source for producing the cultured cell sheet of the present invention are collected from a nasal mucosal tissue. This nasal mucosal tissue may be a mucosal tissue in the nasal cavity, in paranasal cavity, or in mastoid antrum, and examples of the nasal mucosa used in the present invention include an inferior turbinate mucosa, a middle turbinate mucosa, a superior turbinate mucosa, a nasal mucosa, and a paranasal sinus tissue. Among them, an inferior turbinate mucosa is particularly preferable because of its ease of collection. Nasal mucosal tissues used as a source in the present invention are desirable because they are abundant in vivo, and its collection is easy enough to be performed at an outpatient clinic, with little burden on the patients and few ethical issues for the patients.

Cells to be used as a source for producing the cultured cell sheet of the present invention may be, for example, cells themselves collected from a nasal mucosal tissue, cells collected and then cultured and proliferated under culture conditions, or cell strains thereof, but the type of cells is not limited at all.

Animals from which the cells as a source are derived are not particularly limited, including humans, rats, mice, guinea pigs, marmosets, rabbits, dogs, cats, sheep, pigs, goats, monkeys, chimpanzees, and their immunodeficient counterparts. In order to use the cultured cell sheet made from cells collected from a nasal mucosal tissue of the present invention for treatment of human, cells derived from humans, pigs, monkeys, or chimpanzees are preferably used. In order to reduce the side effects associated with cell transplantation such as immune reactions, cells derived from human are particularly preferably used, and autologous cells are most preferably used.

The size of the cultured cell sheet of the present invention can be freely set depending on the site of transplantation. The size of the cultured cell sheet is determined depending on the size of the culture substrate used in the culture. For example, in the cases where a commercially available culture substrate is used, the diameter of the cultured cell sheet can be up to about 35 mm for use of a 6-well plate or a 35-mm dish, and the diameter of the cultured cell sheet can be up to about 60 mm for use of a 60-mm dish. In the case where a cultured cell sheet is prepared using such a plate and used for a transplantation site with a smaller size than that of the cell sheet, the cultured cell sheet can be cut as appropriate for use.

The cultured cell sheet of the present invention may have two or more cultured cell sheets laminated. In another aspect, cell sheets may be laminated in which the cultured cell sheets comprise a mixture of any one or two or more vascular endothelial cells, endothelial progenitor cells, adipose-derived stem cells, and mesenchymal stem cells. The number of laminations is not particularly limited, and should be 10 or less, preferably 5 or less, and more preferably 2 or less. Lamination of the cultured cell sheets made from cells collected from a nasal mucosal tissue can improve the cell density per sheet unit area. The cultured cell sheet made from cells collected from a nasal mucosal tissue of the present invention may also be a mixture of, in addition to mucosal cells, any one or two or more vascular endothelial cells, endothelial progenitor cells, adipose-derived stem cells, and mesenchymal stem cells without impairment of the function of the cell sheet of the present invention as described above. Further, such a cell sheet may be laminated onto a cultured cell sheet made from cells collected from a nasal mucosal tissue.

The cultured cell sheet of the present invention can be produced using any culture substrate, for example, using a culture substrate coated with a temperature-responsive polymer. In the cases where a culture substrate coated with a temperature-responsive polymer is used, mucosal epithelial cells collected from a nasal mucosal tissue can be cultured on a culture substrate coated with a temperature-responsive polymer to prepare a cultured cell sheet on the culture substrate, and then the temperature can be changed to a temperature at which the temperature-responsive polymer has strong hydration force to detach the cultured cell sheet from the culture substrate coated with a temperature-responsive polymer, thereby providing a cultured cell sheet while maintaining the sheet shape. More specifically, a method of producing a cultured cell sheet derived from a nasal mucosal tissue can be provided, the method comprising culturing cells collected from a nasal mucosal tissue on a cell culture substrate coated on its surface with a temperature-responsive polymer having varying hydration force in the temperature range of 0 to 80°C, in a temperature range in which the polymer has weak hydration force, and then changing the temperature of the culture medium to a temperature at which the polymer exhibits high hydration force to detach the cultured cell sheet while keeping the sheet shape.

A cultured cell sheet made from cells collected from a nasal mucosal tissue, prepared using a culture substrate coated with a temperature-responsive polymer in the present invention is not damaged by proteases represented by Dispase, trypsin, and the like during being detached from the cultured cell sheet. Therefore, the cultured cell sheet detached from the culture substrate has adhesive proteins existing in the basal layer when the cell sheet is adhered to the culture substrate. For example, a cultured cell sheet prepared by culturing mucosal cells into a sheet keeps the desmosome structure between cells after being detached. This allows for good adhesion between the cultured cell sheet and the tissue at the transplantation site during transplantation of the cultured cell sheet, which enables efficient transplantation. It is commonly known that Dispase, a protease, enables detachment while keeping 10 to 40% of desmosome structures between cells, but destroys most of basal membrane-like proteins and the like present between the cells and the substrate so that the strength of the resulting cell sheet is reduced. On the other hand, cultured cell sheets made from cells collected from a nasal mucosal tissue prepared by the method of the present invention preserve both desmosome structures and basal membrane-like proteins, and thus can provide various effects as described above.

The cultured cell sheet made from cells collected from a nasal mucosal tissue of the present invention is made from epithelial cells collected from a nasal mucosal tissue. Thus, the cell sheet of the present invention has effects that substitute for the function of a mucosal epithelial tissue, and thus has several characteristic functions, such as facilitating the regeneration of mucosal tissues covering bone tissues. Therefore, transplantation of the cell sheet of the present invention onto the body surface in the middle ear cavity, in the nasal cavity, or in the oral cavity results in facilitation of the regeneration of mucosal tissues covering bone tissues. An object of the present invention is to produce a cell sheet having such functions, and transplant it to a living body to regenerate biological functions. The cultured cell sheet made from cells collected from a nasal mucosal tissue obtained in the present invention may also secrete substances specific for mucosae, such as mucin, hyaluronic acid, and polysaccharides, and the types and amounts of secretion are not particularly limited. Further, a gene may be introduced to the cell sheet used in the present invention in order to improve the function of the cell sheet, and the introduction method preferably follows the usual method.

The cultured cell sheet of the present invention has characteristics as described above, and can be used for use in treatment of disorders developing on the epithelial surface by transplanting it, for example, to the middle ear cavity or the mastoid antrum in the living body. Examples of therapeutic use of the cultured cell sheet of the present invention include, but are not limited to, treatment of diseases such as chronic otitis media, adhesive otitis media, middle ear cholesteatoma, chronic sinusitis, paranasal sinus neoplasm, external auditory canal tumor, external auditory canal atresia, chronic tonsillitis, pharyngeal cancer, laryngeal cancer, oral cancer, tongue cancer, endotracheal granulation, endotracheal cicatrization, tracheal stenosis, tracheal injury, esophageal cancer, vocal cord lesion, inflammatory bowel disease, anal ulcer, anal fissure, hemorrhoid, and anal fistula, and reconstruction of the middle ear cavity after surgery or when other causes require reconstruction of the middle ear cavity.

### <Method of producing cultured cell sheet>

In one aspect of the present invention, a method of producing the above-mentioned cultured cell sheet of the present invention can be provided. Specifically, a method of producing a cultured cell sheet made from cells collected from a nasal mucosal tissue as a source is provided, comprising the steps of: (1) preparing mucosal epithelial cells as the source from the nasal mucosal tissue; (2) culturing the prepared mucosal epithelial cells to increase the number of the mucosal epithelial cells as the source; (3) culturing the obtained cells on a cell culture substrate to prepare a cultured cell sheet in which the cells cover the culture substrate surface on the culture substrate; and (4) detaching and collecting the cultured cell sheet prepared on the culture substrate while maintaining the sheet shape.

### Step (1): Collection of source cells from tissue

A cultured cell sheet according to the present invention is made from cells collected from a nasal mucosal tissue. Thus, in the present invention, tissues collected from within the mucosa in the nasal cavity, paranasal cavity, or mastoid antrum are required to be made into cells in a separated (discrete) state by a specific process. In this case, the process method may be, for example, an explant culture method in which a nasal mucosal tissue collected from a subject is left to stand directly on a cell culture substrate without enzyme treatment, and cultured in serum-containing medium or serum-free medium to allow the cells in the tissue to migrate.

The medium used for the explant culture in the step may be any of serum-containing medium or medium not containing serum (so-called serum-free medium), and may be performed as appropriate depending on the cells used according to the conventional method, which is not particularly limited. For example, in the cases where serum-containing medium is used, the base medium may be, for example, DMEM medium, F-12 medium, KCM medium, or a mixture thereof. In this case, additives as commonly used in cell culture, such as serum, may be added to the medium. Alternatively, in cases where serum-free medium is used, examples of the serum-free medium that may be used include various commercially available serum-free culture media for epithelial cells such as UltraCULTURE serum-free medium (Lonza), PC-1 serum-free medium (Lonza), EpiCult Medium (VERITAS), EpiLife Medium (ThermoFisher). In this case, additives as commonly used in serum-free cell culture, such as Supplement S7 (Gibco) or human serum albumin, may be added to the medium.

In the cases where serum is added to the medium used in this step, fetal bovine serum or human serum can be used. In this case, the concentration of the serum used in the medium is 0.5 to 35%, preferably 1 to 20%, more preferably 5 to 15%, most preferably 8 to 12%.

In this step, in order to prevent cell death (apoptosis) of nasal mucosal epithelial cells migrating from a nasal mucosal tissue, a ROCK (Rho-associated kinase) inhibitor can be added to the culture medium. Examples of the ROCK inhibitor that can be used in the present invention include, but are not limited to, Y-27632, ripasudil hydrochloride hydrate (Kowa Company, Ltd.), Thiazovivin, Fasudil, GSK429286A, RKI-1447, H-1152, and Azaindole 1.

A cell culture substrate with its surface previously coated with collagen, laminin, fibronectin, Matrigel, serum and a mixture thereof as necessary to increase the adhesion to cells may be used. Alternatively, a cell culture substrate with the surface positively charged may be used. However, the present invention is not particularly limited to these conditions.

In this step, the number of days for explant culture can be determined as appropriate while monitoring over time the shape and number of cells migrating from the nasal mucosal tissue to the culture medium. As usual, cells are collected after performing explant culture for 10 to 14 days, and confirming that cells migrate and cover 70% of the culture surface of the culture substrate, but the number of days of explant culture can be extended or shortened as monitoring, for example, the culture conditions.

For obtaining nasal cavity epithelial cells that have migrated in explant culture, nasal cavity epithelial cells attached to the cell culture substrate are collected by enzymatically treating the nasal cavity epithelial cells to allow the nasal cavity epithelial cells to float off the cell culture substrate. In this case, an enzyme such as trypsin, protease, or collagenase can be used.

Examples of culture substrates that can be used in the step (1) of the present invention include dish type culture substrates, flask type culture substrates, and multiwell type culture substrates. The material of the cell culture substrate to be coated may be any material, including compounds commonly used in cell culture, such as glass, modified glass, polystyrene, and polymethyl methacrylate, and materials that can generally be shaped, such as polymer compounds except for those described above, and ceramics.

### Step (2): Cell proliferation

The number of the epithelial cells as a source obtained in the step (1) may be insufficient by themselves for the subsequent culture. Thus, optionally as a step (2), mucosal epithelial cells prepared in the step (1) can be cultured to increase the number of mucosal epithelial cells as a source. It is noted that the passage number can be determined based on the relationship between the number of cells obtained in the step (1) and the desired number of cells, but increasing the number of passages generally changes cell properties, and thus the passage number is preferably as low as possible. Therefore, in the cases where a sufficient number of cells for culturing of cells into sheets in the step (3) as described below is obtained in the step (1), the step (2) need not be performed.

Similarly as in the steps (1-a) and (1-b), the step (2) can include addition of serum to the culture medium, and addition of a ROCK (Rho-associated kinase) inhibitor to the culture medium in order to prevent cell death (apoptosis) of nasal mucosal epithelial cells during culture.

The culture substrate that can be used in step (2) of the present invention can be the same as in the step (1). Similarly as in the step (1), the culture medium that can be used in the step (2) may also be serum-containing medium or serum-free medium.

### Step (3): Cell culturing into sheets

According to the present invention, thus-obtained cells are seeded onto a cell culture substrate and cultured to prepare a cell sheet in which the cells cover the surface of the cell culture substrate on the cell culture substrate. In this case, the number of cells to be seeded is usually preferably from 3.0 × 10⁴ to 2.0 × 10⁵ cells/cm², preferably from 4.0 × 10⁴ to 1.5 × 10⁵ cells/cm², more preferably from 5.0 × 10⁴ to 1.0 × 10⁵ cells/cm². However, a seeding concentration of less than 3.0 × 10⁴ cells/cm² results in poor proliferation of mucosal cells, and worsened manifestation of the function of the resulting cultured cell sheet derived from a nasal mucosal tissue, which is not preferred for implementation of the present invention. Conversely, the seeding cell number of more than 2.0 × 10⁵ cells/cm² results in worsened proliferation of mucosal cells, which is not preferred.

A cell culture substrate used in this step according to the present invention can be a cell culture substrate coated on its surface with a temperature-responsive polymer. In the cases where a cell culture substrate coated on its surface with a temperature-responsive polymer is used as a cell culture substrate, the step (3) can be performed by culturing the prepared mucosal epithelial cells on a cell culture substrate coated on its surface with a temperature-responsive polymer having varying hydration force in the temperature range of 0 to 80°C, in a temperature range in which the temperature-responsive polymer has weak hydration force to prepare a cultured cell sheet on the cell culture substrate.

The medium used for the cell culture according to the present invention may be any of serum-containing medium or medium not containing serum (so-called serum-free medium), and may be performed as appropriate depending on the cells used according to the conventional method, which is not particularly limited. For example, for nasal mucosal epithelial cells, KCM medium, DMEM medium, F-12 medium, or a mixture thereof may be used. In this case, additives as commonly used in cell culture, such as serum, may be added to the medium. Alternatively, various commercially available serum-free culture media for epithelial cells may be used, such as UltraCULTURE serum-free medium (Lonza), PC-1 serum-free medium (Lonza), EpiCult Medium (VERITAS), and EpiLife Medium (ThermoFisher), which are commercially available as serum-free media. In the cases where a serum-containing medium is used, a serum such as fetal bovine serum (FBS), human allogeneic serum, or human autoserum can be used. Preferably, human autoserum is used from the viewpoint of the proliferative ability of cells and immune reaction in vivo after transplantation of the cultured cell sheet.

Feeder cells may be used in preparation of the cultured cell sheet of the present invention in order to facilitate the cell proliferation and cell sheet formation of nasal mucosal epithelial cells. In this case, the cultured cell sheet may be prepared by mixing feeder cells and mucosal epithelial cells as a source and seeding the mixture onto a cell culture substrate, or can also be prepared by first culturing feeder cells in a cell culture substrate to allow them to form a monolayer, and seeding mucosal epithelial cells as a source onto the monolayer. In the cases where feeder cells are used to prepare a cultured cell sheet, feeder cells such as mouse-derived fibroblasts, mouse embryonic fibroblasts, or autologous cells treated with gamma irradiation or antibiotics, more specifically 3T3-J2 cells can be used. Feeder cells can elicit an immune reaction in vivo after transplantation, but the effect is minimal because they are eliminated to the surface during culture.

On the other hand, the cultured cell sheet of the present invention can be prepared without using feeder cells by seeding source cells onto a cell culture substrate, culturing them in a serum-free medium until full confluency is achieved, then replacing the serum-free medium with a serum-containing medium or the like, and culturing and piled up them for 4 to 7 days. This is preferable because there is less risk in terms of the immune response that may occur in vivo after transplantation.

The present inventors have found that addition of human serum during culture of human nasal mucosal epithelial cells regardless of the shape of the culture substrate as described later leads to good proliferation of the cultured human nasal mucosal epithelial cells, resulting in obtaining a human nasal mucosal epithelial cell sheet with physical strength and flexibility. In the case where human serum is used, the serum used for the case can also be serum from other people, but use of autoserum from the patient from which the nasal mucosal tissue has been collected results in much higher cell proliferation, as well as obtaining a human nasal mucosal epithelial cell sheet in good condition that is physically strong and flexible and thus is relatively preferable for the present invention. Here, if a serum is used, the concentration of the serum used in the medium is desirably 0.5 to 35%, preferably 1 to 20%, more preferably 5 to 15%, and most preferably 8 to 12%. A serum concentration more than 30% can result in reduced strength of the cultured cells and thus is undesirable for the present invention.

The shape of the culture substrate for the step (3) in the present invention is preferably tabular. In the cases where culture substrates having such a shape are used for cell culture, the resulting cultured cell sheets of the present invention can be easily detached from the culture substrates in the step (4) described later, and in addition, structures comprising basal structures are likely to remain after detachment so that the cell sheets are physically strong and are also flexible.

In order to increase the cell adhesion onto the surface of the culture substrate to be used, various extracellular matrix (ECM), serum, or a combination thereof can also be used as appropriate to coat the surface regardless of the presence of treatment with a temperature-responsive polymer. As ECM, type I collagen, type IV collagen, laminin, fibronectin, Matrigel, poly-D-lysine, and the like, as well as a combination thereof may be used. However, the present invention is not particularly limited to these conditions.

In the step (3) according to the present invention, the mucosal epithelial cells prepared through the step (1) and step (2) are cultured on a cell culture substrate coated on its surface with a temperature-responsive polymer having varying hydration force in the temperature range of 0 to 80°C, in a temperature range in which the temperature-responsive polymer has weak hydration force. In general, the temperature at which the temperature-responsive polymer has a weak hydration force is preferably 37°C, a temperature for culturing cells. The temperature-responsive polymer used in the present invention may be either a homopolymer or a copolymer. Examples of such a polymer include polymers described in Japanese Patent Application Laid-Open No. H2-211865. Specifically, for example, temperature-responsive polymers used in the present invention are obtained by homopolymerization or copolymerization of monomers as described below. Examples of monomers that can be used include (meth)acrylamide compounds, N- (or N,N-di)alkyl substituted (meth)acrylamide derivatives, and vinyl ether derivatives. Any two or more of them can be used for copolymers. In addition, copolymerization with monomers other than the monomers described above, graft polymerization or copolymerization between polymers, or mixtures of polymers and copolymers may be used. The polymers can also be crosslinked without impairing the nature of the polymers. In this case, separation is performed in the range of 5°C to 50°C since those to be cultured and detached are cells. Thus, the temperature-responsive polymer may be, for example, poly-(n-propylacrylamide) (the lower critical solution temperature of the homopolymer is 21°C), poly-(N-n-propylmethacrylamide) (the above-described temperature is 27°C), poly-(N-isopropylacrylamide) (the above-described temperature is 32°C), poly-(N-isopropylmethacrylamide) (the above-described temperature is 43°C), poly-(N-cyclopropylacrylamide) (the above-described temperature is 45°C), poly-(N-ethoxyethylacrylamide) (the above-described temperature is about 35°C), poly-(N-ethoxyethylmethacrylamide) (the above-described temperature is about 45°C), poly-(N-tetrahydrofurfurylacrylamide) (the above-described temperature is about 28°C), poly-(N-tetrahydrofurfuryl methacrylamide) (the above-described temperature is about 35°C), poly-(N,N-ethylmethylacrylamide) (the above-described temperature is 56°C), or poly-(N,N-diethylacrylamide) (the above-described temperature is 32°C). Examples of monomers for copolymerization used to produce temperature-responsive polymers used in the present invention include, but not particularly limited to, hydrated polymers such as polyacrylamide, poly-(N,N-diethylacrylamide), poly-(N,N-dimethylacrylamide), polyethylene oxide, polyacrylic acids and salts thereof, polyhydroxyethylmethacrylate, polyhydroxyethylacrylate, polyvinyl alcohols, polyvinylpyrrolidone, cellulose, and carboxymethylcellulose.

The method of coating the surface of substrates with temperature-responsive polymers as described above used in the step (3) in the present invention is not particularly limited. For example, the method can be performed, for example, by either electron beam (EB), gamma ray irradiation, ultraviolet irradiation, plasma treatment, cona treatment, or organic polymerization reaction, or by physical adsorption such as application or kneading, for substrates and temperature-responsive polymers as described above. The amount of a temperature-responsive polymer coating on the surface of the culture substrate is desirably in the range of 1.1 to 2.3 µg/cm², preferably 1.4 to 1.9 µg/cm², and more preferably 1.5 to 1.8 µg/cm². In the cases where the coating amount is less than 1.1 µg/cm², the cells on the polymer are difficult to be detached even when stimulated, resulting in significantly reduced operating efficiency, which is undesirable. On the other hand, in the cases where the coating amount is 2.3 µg/cm² or more, it is difficult for cells to adhere to the region, resulting in difficulty in allowing sufficient cells to adhere. In such a case, the amount of a temperature-responsive polymer coating on the surface of a substrate may be 2.3 µg/cm² or more if a cell-adhesive protein is further coated onto the temperature-responsive polymer coating layer. In this case, the coating amount of the temperature-responsive polymer is desirably 9.0 µg/cm² or less, preferably 8.0 µg/cm² or less, and conveniently 7.0 µg/cm² or less. In the cases where the amount of a temperature-responsive polymer coated is 9.0 µg/cm² or more, cell attachment is more difficult even when a cell-adhesive protein is coated on the temperature-responsive polymer coating layer, which is undesirable. The type of such a cell-adhesive protein is not particularly limited, and may be, for example, collagen, laminin, laminin 5, fibronectin, or Matrigel alone, or a mixture of two or more of them. The method of applying the cell-adhesive protein may according to the usual method, and in usual a method in which an aqueous solution of a cell-adhesive protein is applied to the surface of the substrate, followed by removal of the aqueous solution and rinsing is used. The step (3) in the present invention is a step using a technology for utilizing a cell sheet itself using a cell culture substrate coated with a temperature-responsive polymer. Therefore, it is not desirable for the amount of the cell-adhesive proteincoated on the temperature-responsive polymer layer to be extremely high. The measurement of the amount of the applied temperature-responsive polymer and the amount of the applied cell-adhesive protein may be performed according to the usual methods, including, for example, methods in which the cell adhesion area is directly analyzed using FT-IR-ATR; and methods in which a pre-labeled polymer is immobilized in the same manner and inferences are made based on the amount of the labeled polymer immobilized on the cell adhesion area, any of which may be used.

### Step (4): Detachment of cell sheet from cell culture substrate

In accordance with the present invention, a cultured cell sheet made from cells collected from a nasal mucosal tissue can be obtained in a step in which a cultured cell sheet prepared on a cell culture substrate in the step (3) is detached and collected while keeping the sheet shape (step (4)). In the cases where a cell culture substrate coated on its surface with a temperature-responsive polymer is used as a cell culture substrate, this step (4) is performed by changing the temperature of the culture medium into a temperature at which the temperature-responsive polymer has a strong hydration force to detach the cultured cell sheet from the cell culture substrate and obtain the cultured cell sheet while maintaining the sheet shape.

In order to detach and collect a cultured cell sheet from a culture substrate coated with a temperature-responsive polymer in the step (4) method in the present invention, detachment can be made by changing the temperature of the culture substrate to which the cultured cells attach to a temperature not lower than the upper limit critical solution temperature or not higher than the lower critical solution temperature of the coating polymer on the culture substrate. This can be performed in culture media or in other isotonic solutions, which can be chosen according to the purpose. In order to detach and collect cells more rapidly and efficiently, for example, methods comprising tapping or shaking substrates lightly; methods further comprising agitating media using a pipette; methods comprising detaching cells using tweezers; methods comprising detaching cells using a cell scraper; methods comprising detaching cells by adding media at 4°C; methods comprising detaching cells after leaving them on ice; methods comprising detaching cells after leaving them at 20°C; and methods comprising detaching cells using carriers for cultured cell sheets may be used alone or in combination.

The methods described above will be described using poly(N-isopropylacrylamide) as an example of a temperature-responsive polymer. Poly(N-isopropylacrylamide) is known to be a polymer having a lower critical solution temperature of 31°C. The polymer, when in a free state, is dehydrated in water at a temperature of 31°C or higher, so that the polymer chains are aggregated and cause cloudiness. Conversely, at a temperature of 31°C or lower, the polymer chains are hydrated and dissolved in water. In the present invention, this polymer is coated and fixed on the surface of a substrate such as a dish. Thus, the polymer on the surface of the substrate is dehydrated at a temperature of 31°C or higher as described above. Since the polymer chains are coated and fixed on the surface of the substrate, the surface of the substrate will exhibit hydrophobic properties. Conversely, the polymer on the surface of the substrate is hydrated at a temperature of 31°C or lower. Since the polymer chains are coated and fixed on the surface of the substrate, the surface of the substrate will exhibit hydrophilic properties. In such cases, the hydrophobic surface properly allows cells to adhere thereto and grow thereon, while the hydrophilic surface is such a surface to which cells cannot adhere and from which cells or cell sheets during culture are detached simply by being cooled.

In order to detach and collect a cultured cell sheet made from cells collected from a nasal mucosal tissue from a culture substrate in the step (4) method in the present invention, a carrier may be closely adhered on the cultured cells at the end of culture as necessary, and then the cultured cell sheet made from cells collected from a nasal mucosal tissue may be collected together with the carrier. Typically, in order to detach and collect a cultured cell sheet from a culture substrate coated with a temperature-responsive polymer in the step (4), a carrier can be closely adhered on the cultured cells at the end of culture, and then the temperature is changed to not lower than the upper limit critical solution temperature or not higher than the lower critical solution temperature of the coating polymer on the culture substrate to detach the cultured cell sheet made from cells collected from a nasal mucosal tissue together with the carrier.

The material of the carrier used as necessary may be, for example, hydrophilizated polyvinylidenedifluoride (PVDF), polypropylene, polyethylene, polylactic acid, cellulose or a derivative thereof, chitin, chitosan, collagen, paper such as Japanese paper, urethane, or a net or stockinette polymer material such as spandex.

In the present invention, two or more cultured cell sheets made from cells collected from a nasal mucosal tissue collected in the step (4) can be used in a laminated state. The method of laminating cultured cell sheets according to the present invention is not particularly limited, and can be achieved, for example, by detaching cultured cell sheets while keeping the sheet shape, and laminating the detached cultured cell sheets using a jig for transferring cultured cells as necessary. For lamination of cultured cell sheets, detached cultured cell sheets can be cultured under usual culture conditions used when the cultured cell sheets are prepared to allow them to adhere to each other. The medium used may be, for example, a medium supplemented with a serum, such as human serum or fetal bovine serum (FBS), or a serum-free medium without addition of any serum. A jig for transferring cell sheets may also be optionally used. Such a jig may have any material and shape without any limitation so long as it can capture a detached cell sheet. Examples of the material typically include polyvinylidenedifluoride (PVDF), silicon, polyvinyl alcohol, polylactic acid, urethane, cellulose and derivatives thereof, chitin, chitosan, collagen, gelatin, and fibrin glue. Examples of the shape include membrane, porous film, nonwoven fabric, and woven fabric. Such a jig can be used in contact with a cultured cell sheet.

A thus-produced cultured cell sheet of the present invention can be transplanted to a desired site in vivo. Examples of such a transplantation site include mucosa-defective sites in the middle ear cavity, in the mastoid antrum, in the nasal cavity, and in the oral cavity. Among them, in particular, transplantation at a mucosa-defective site in the middle ear cavity is a transplantation site where the present invention can be expected to be effective.

The state of the transplantation site is not particularly limited, and vascular induction may be performed in advance, or fibrin glue, collagen, or other intercellular adhesive components may be applied in order to enhance the engraftment of the cultured cell sheet to the transplantation site. Here, for example, in the cases where vascular induction is performed before transplantation, the method of performing vascular induction on transplantation sites is not also particularly limited. Examples of the method include a method in which FGF, a vascular growth factor, is encapsulated in microspheres and allowed to act on an organism for 8 to 10 days while changing the composition, size, and injection area of the microspheres, and a method comprising cutting a polyethylene terephthalate mesh to any size, creating a bag shape, placing FGF dissolved in a high concentration agarose solution inside the bag, and removing the bag after 8 to 10 days to create a vascular induced space.

The present invention will be described in more detail with reference to Examples which are not meant to limit the scope of the present invention by no means. The following Examples have been basically conducted with the permission of the institutional ethical committee.

### EXAMPLES

### Example 1: Preparation of source cells by serum-containing explant culture method

In this Example, mucosal tissues were collected from inferior turbinate mucosae in the nasal cavity of surgical patients using an endoscope, from which source cells for preparation of cultured cell sheets were then prepared by a serum-containing explant culture method.

The collected tissues were immersed in an Isodine solution for disinfection, and then sliced such that the thickness of the interstitial tissue layer was equal to the thickness of the epithelial tissue layer (the ratio between the thicknesses is about 1.0). Thereafter, the tissues were cut into 1-mm to 2-mm square sections, and then without enzymatic treatment, four pieces of the section were left to stand on one Primaria (Corning) culture substrate. After culturing for 5 days in a KCM medium or a FAD medium (both culture media containing DMEM medium: Ham's F12 = 3:1, supplemented with 10% FBS and a ROCK inhibitor (10 µM, Y-27632 (WAKO))), the culture medium was replaced with a KCM medium supplemented with 10% FBS and a ROCK inhibitor. Thereafter, at days 8 and 11, explant culture was performed in which cells contained in the tissues were allowed to migrate while the culture medium was replaced with the above-described medium. The culturing was continued until the migrating cells occupied about 70% of the surface of the cell culture substrate.

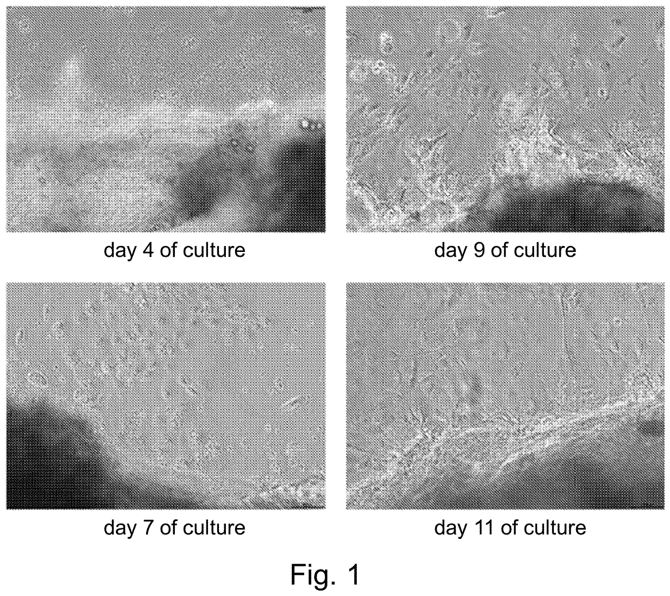

This resulted in migration of fibroblast-like cells and undifferentiated cells from the nasal mucosal tissues (FIG. 1). The migrating cells were collected by treating them with trypsin and detaching them from the cell culture substrate. The number of the cells was 34.3 × 10⁴ cells/dish.

### Example 2: Preparation of mucosal epithelial cell sheet by serum-containing culture using feeder cells

In this Example, mucosal epithelial cell sheets were prepared using the cells obtained in Example 1 as a source under serum-containing culture conditions using feeder cells.

The feeder cells used were mouse-derived 3T3-J2 cells (donated by J-TEC), and proliferated by passage culture using a KCM medium containing a ROCK inhibitor (10 µM, Y-27632 (WAKO)). The cultured and proliferated cells were collected via trypsin treatment, and used to prepare cell suspensions.

Next, 1 to 2 × 10⁴ cells/cm² of the cells obtained by the explant culture method in Example 1 and the feeder cells were mixed, and the mixture was seeded onto a cell culture substrate (Corning Primaria) and cultured using a KCM medium supplemented with a ROCK inhibitor (10 µM Y-27632 (WAKO)) for 4 days. The cultured and proliferated nasal mucosal epithelial cells were collected via trypsin treatment, and used to prepare cell suspensions with the cell density adjusted to 26.4 × 10⁴ cells/mL.

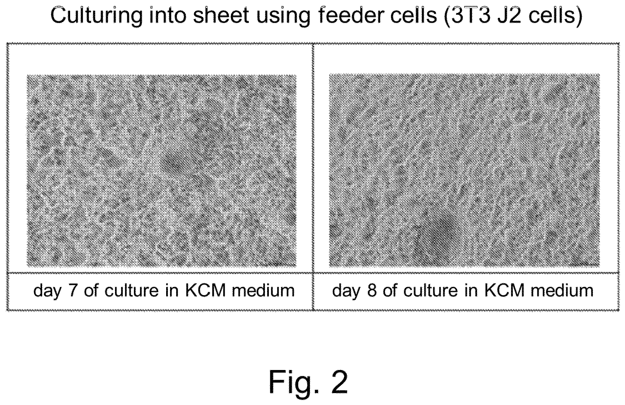

The nasal mucosal epithelial cells prepared as described above were mixed with feeder cells, and this mixture was seeded onto two cell culture substrates (35 mm, CellSeed Inc.) coated with a temperature-responsive polymer (poly-N-isopropylacrylamide). As a comparator, the nasal mucosal epithelial cells prepared as described above were seeded onto cell inserts coated with a temperature-responsive polymer used in Patent Document 2. In both cases, the seeding densities of the nasal mucosal epithelial cells were 3 × 10⁴ cells/cm² for the cell culture substrates coated with a temperature-responsive polymer and 3 × 10⁴ cells/cm² for the cell inserts coated with a temperature-responsive polymer. In both cases, the medium used was the same KCM medium (containing 10% FBS and a ROCK inhibitor (10 µM, Y-27632 (WAKO))) as in the primary culture in Example 1. While replacing the culture medium with a KCM medium not containing a ROCK inhibitor at days 3, 5, and 7 of culturing, the culturing was continued for 8 days to prepare sheet-shaped cells on the culture substrate (FIG. 2). Thereafter, the temperature of the culture media was changed to 20°C to detach a nasal mucosal cell sheet from each culture substrate coated with a temperature-responsive polymer.

The obtained cell sheets were compared for their strength. The cell sheets collected from the cell culture substrates coated with a temperature-responsive polymer were successfully detached while being grasped with tweezers, while those obtained from the cell inserts coated with a temperature-responsive polymer had such a collapsed structure that they were not successfully grasped with tweezers, demonstrating that the cell sheets collected from the cell culture substrates coated with a temperature-responsive polymer were stronger than those obtained from the cell inserts coated with a temperature-responsive polymer.

In addition, with respect to the detachment of the cell sheets, ease of detachment, or time to detachment when using the cell culture substrate coated with a temperature-responsive polymer was significantly shorter than that when using a cell insert coated with a temperature-responsive polymer.

The cell culture substrate coated with a temperature-responsive polymer and the cell insert coated with a temperature-responsive polymer did not show any significant difference in the viable cell count, cell survival, and positive rate for pan-cytokeratins.

Next, the cell composition of the obtained cell sheets was examined by immunohistochemical staining. The subjects detected were mouse-derived feeder cells; undifferentiated cells expressing p63 that is a marker for epithelial stem/ progenitor cell; and fibroblasts. The cell sheets were prepared using nasal mucosal tissues collected from four human subjects (subject IDs: #1, #2, #3, and #4) as sources, and using a cell culture substrate coated with a temperature-responsive polymer. The detached cell sheets were fixed with paraformaldehyde, then embedded in paraffin, and cut thinly in the vertical direction such that the cross section of the cultured cell sheet can be observed to prepare tissue sections. In a total of 32 tissue sections per cell sheet in which the locations were randomly selected, mouse-derived feeder cells, undifferentiated cells expressing p63 that is a marker for epithelial stem/ progenitor cell, and fibroblasts were detected.

Mouse-derived feeder cells were detected by immunohistochemical staining using an anti-rabbit PDGFRα antibody (Cell Signaling).

Undifferentiated cells expressing p63 were detected by reacting the tissue sections with an anti-p63 antibody (Abcam) as a primary antibody, and developing and observing the reaction products using EnVision (DAKO) and DAB (DAKO).

Fibroblasts were detected by reacting cells with an antibody (Cell Signaling) against platelet-derived growth factor receptor α (PDGFRα) that is a fibroblast marker as a primary antibody, and developing and observing the reaction products using EnVision (DAKO) and DAB (DAKO).

Ninety-six fields of view on the tissue sections were observed for mouse-derived feeder cells, which were not found to be present.

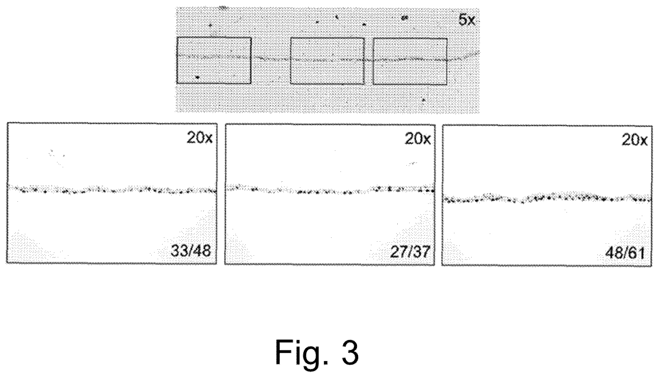

Next, three fields of view on each of 32 tissue sections (96 in total) were observed for the expression of p63 by immunohistochemical staining. Specific observations are shown in FIG. 3. The numbers (33/48, 27/37, and 48/61) at the bottom right of the images at 20x magnification (20X) represent 33, 27, and 48 p63-positive cells out of a total of 48, 37, and 61 cells in the fields of view, respectively. Measurement on all fields of view (96 per subject) resulted in 65 to 82% of undifferentiated cells contained relative to the total number of cells within each field of view for the cell sheets prepared using cell culture substrates coated with a temperature-responsive polymer (see, table 1).

[Table 1]

**Table 1**

| Subject ID | p63 positive rate (%) | SD |
|---|---|---|
| #1 | 71.28 | 5.39 |
| #2 | 66.67 | 4.60 |
| #3 | 65.38 | 4.47 |
| #4 | 81.84 | 3.64 |
| Overall | 71.29 | 7.92 |

On the other hand, the percentage of fibroblasts with respect to the obtained cell sheet was determined by immunohistochemical staining, the cell sheets prepared using a cell culture substrate coated with a temperature-responsive polymer showed only 0 to 1 PDGFRα-positive cell (i.e., fibroblast) per field of view with respect to the total number of cells in each field of view (the mean number of cells among all fields of view in all subjects = 34.51). The fibroblast percentages were in the range of 0 to 2.9%.

From the above, use of a cell culture substrate in a dish shape coated with a temperature-responsive polymer to produce a nasal mucosal cell sheet on the cell culture substrate was found to result in higher strength than those obtained by conventional art and thus excellent structure.

### Example 3: Preparation of mucosal epithelial cell sheet by serum-containing culture without using feeder cells

In this Example, mucosal epithelial cell sheets were prepared using the cells obtained in Example 1 as a source under serum-containing culture conditions without using feeder cells.

The cells obtained by the explant culture method in Example 1 were seeded onto a cell culture substrate (Corning Primaria) and cultured using a KCM medium supplemented with a ROCK inhibitor (10 µM Y-27632 (WAKO)) for 4 days.

The cultured and proliferated cells were collected via trypsin treatment, and used to prepare cell suspensions with the cell density adjusted to 19 × 10⁴ cells/ml, which were then seeded onto two cell culture substrates coated with a temperature-responsive polymer (poly-N-isopropylacrylamide) (35 mm, Cell Seed Inc.). As a comparator, the cells were similarly seeded onto cell inserts coated with a temperature-responsive polymer used in Patent Document 2. In both cases, the seeding densities were 10 × 10⁴ cells/cm² for the cell culture substrates coated with a temperature-responsive polymer and 20 × 10⁴ cells/cm² for the cell inserts coated with a temperature-responsive polymer. The medium used was the same KCM medium (containing 5% FBS) supplemented with a ROCK inhibitor (10 µM, Y-27632 (WAKO)) as in the primary culture in Example 1. While replacing the culture medium with a KCM medium not containing a ROCK inhibitor at days 3, 5, and 7 of culturing, the culturing was continued for 8 days to prepare sheet-shaped cells on the culture substrate. Thereafter, the temperature of the culture media was changed to 20°C to detach a nasal mucosal cell sheet from each culture substrate coated with a temperature-responsive polymer.

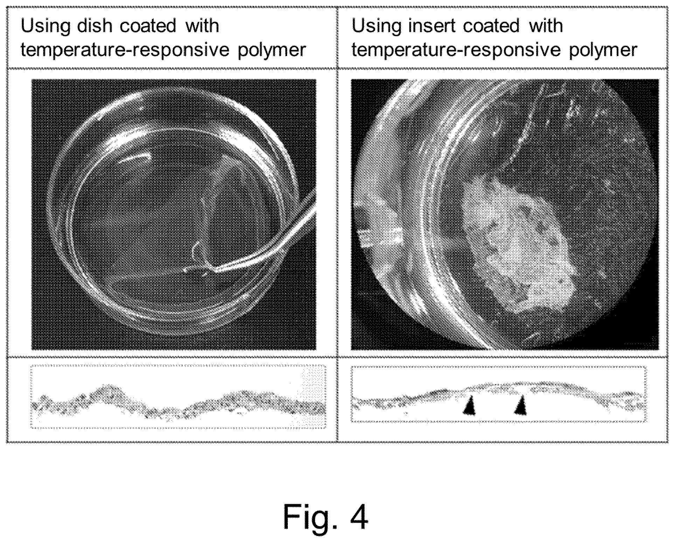

The obtained cell sheets were compared for their strength. The cell sheets collected from the cell culture substrates coated with a temperature-responsive polymer were successfully detached while being grasped with tweezers, while those obtained from the cell inserts coated with a temperature-responsive polymer had such a collapsed structure that they were not successfully grasped with tweezers (FIG. 4), demonstrating that the cell sheets collected from the cell culture substrates coated with a temperature-responsive polymer were stronger than those obtained from the cell inserts coated with a temperature-responsive polymer.

In addition, with respect to the detachment of the cell sheets, ease of detachment, or time to detachment when using the cell culture substrate coated with a temperature-responsive polymer was significantly shorter than that when using a cell insert coated with a temperature-responsive polymer.

The cell culture substrate coated with a temperature-responsive polymer and the cell insert coated with a temperature-responsive polymer did not show any significant difference in the viable cell count, cell survival, and positive rate for pan-cytokeratins.

Next, the cell composition of the obtained cell sheets was examined by immunohistochemical staining. The subjects detected were undifferentiated cells expressing p63 that is a marker for epithelial stem/ progenitor cell; and fibroblasts. The cell sheets were prepared using nasal mucosal tissues collected from three human subjects (subject IDs: #5, #6, and #7) as sources, and using a cell culture substrate coated with a temperature-responsive polymer. Undifferentiated cells expressing p63 and fibroblasts were detected by the methods described in Example 2.

Determination of the expression of p63 by immunohistochemical staining in three fields of view on each of 32 tissue sections (96 in total) resulted in about 64 to 81% of undifferentiated cells contained relative to the total number of cells within each field of view for the cell sheets prepared using cell culture substrates coated with a temperature-responsive polymer (see, Table 2).

[Table 2]

**Table 2**

| Subject ID | p63 positive rate (%) | SD |
|---|---|---|
| #5 | 64.75 | 8.85 |
| #6 | 72.73 | 6.78 |
| #7 | 80.90 | 2.84 |
| Overall | 72.79 | 9.36 |

On the other hand, the percentage of fibroblasts with respect to the obtained cell sheet was determined by immunohistochemical staining, the cell sheets prepared using a cell culture substrate coated with a temperature-responsive polymer showed only 0 to 3 of PDGFRα-positive cells (i.e., fibroblast) per field of view with respect to the total number of cells in each field of view (the mean number of cells among all fields of view in all subjects = 70.05). The fibroblast percentages were in the range of 0 to 4.3%.

From the above, use of a cell culture substrate in a dish shape coated with a temperature-responsive polymer to produce a nasal mucosal cell sheet on the cell culture substrate was found to result in higher strength than those obtained by conventional art and thus excellent structure.

### Example 4: Preparation of source cells by explant culture method using serum-free medium

In this Example, mucosal tissues were collected from inferior turbinate mucosae in the nasal cavity of surgical patients using an endoscope, from which source cells for preparation of cultured cell sheets were then prepared by an explant culture method using a medium in which EpiLife Medium (ThermoFisher) is supplemented with Supplement S7 (ThermoFisher).

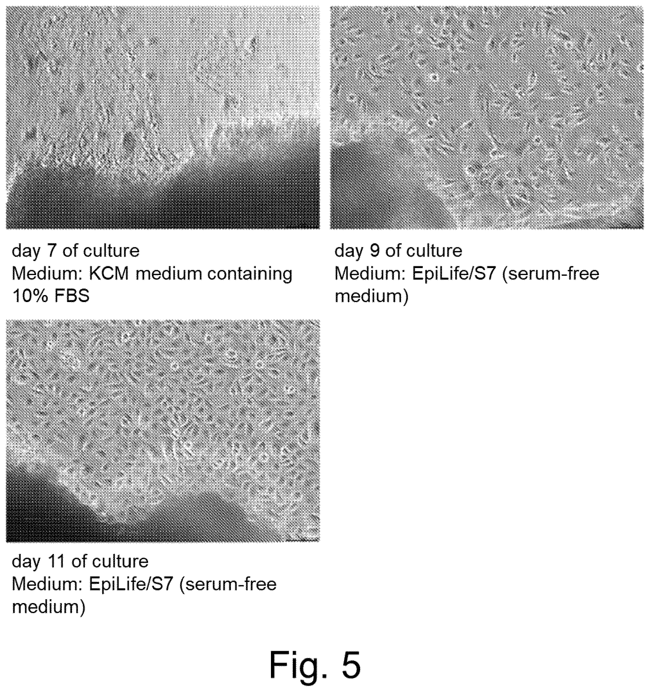

The collected tissues were immersed in an Isodine solution for disinfection, and then sliced such that the thickness of the interstitial tissue layer was equal to the thickness of the epithelial tissue layer (the ratio between the thicknesses is about 1.0). Thereafter, the tissues were cut into 1-mm to 2-mm square sections, and then without enzymatic treatment, two to three pieces of the section were left to stand in 1 well of TrueLine Cell Culture plate 6-well. In a KCM medium or FAD medium (both culture media containing DMEM medium: Ham's F12 = 3:1 not supplemented with any ROCK inhibitor and supplemented with 10% FBS), the cell migration was observed (FIG. 5, at day 7 of culture).

Thereafter, the culture medium was replaced with a serum-free medium (medium in which EpiLife Medium (Thermo Fisher) was supplemented with Supplement S7 (Thermo Fisher)), and then explant culture was performed while replacing the culture medium with the serum-free medium every 1 to 2 days. The culture was continued for 11 days until the migrating cells occupied about 70% of the surface of the cell culture substrate.

This resulted in migration of fibroblast-like cells and undifferentiated cells from the nasal mucosal tissues (FIG. 5, at day 11 of culture). The migrating cells were collected by performing enzymatic treatment for recombinant cell detachment and detaching them from the cell culture substrate. The number of the cells was 2.7 × 10⁵ cells/dish.

### Example 5: Preparation of mucosal epithelial cell sheet by culture using EpiLife Medium and without using feeder cells

In this Example, mucosal epithelial cell sheets were prepared using the cells obtained in Example 4 as a source without using feeder cells.

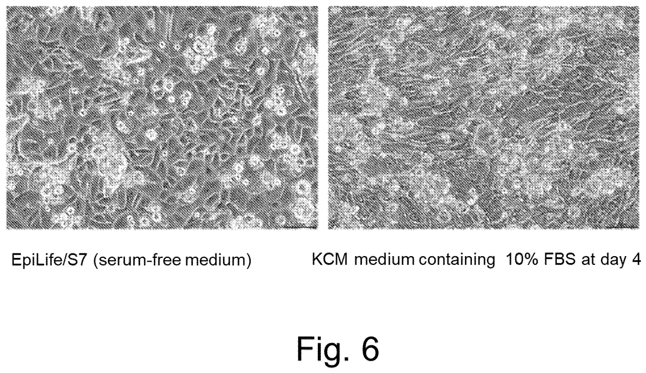

2,000 to 5,000 cells/cm² of the cells obtained in the explant culture method in Example 4 were seeded onto a polystyrene Cell Culture dish (Greiner Bio-One) cell culture substrate, and cultured in a serum-free medium (medium in which EpiLife Medium (Thermo Fisher) was supplemented with Supplement S7 (Thermo Fisher)) for 6 days until confluency was reached (FIG. 6, left). The cultured and proliferated nasal mucosal epithelial cells were collected via enzymatic treatment for detachment of recombinant cells, and used to prepare cell suspensions.

The nasal mucosal epithelial cells prepared as described above were seeded onto two cell culture substrates (35 mm, CellSeed Inc.) coated with a temperature-responsive polymer (poly-N-isopropylacrylamide). The seeding density of the nasal mucosal epithelial cells was 3 × 10⁴ cells/cm². The medium used was a serum-free medium (medium in which EpiLife Medium (Thermo Fisher) was supplemented with Supplement S7 (Thermo Fisher)). The culture was continued for 7 days until full confluency was achieved, with medium exchange every 1 to 2 days. Thereafter, the medium was replaced with a KCM medium or a FAD medium (both culture media containing DMEM medium: Ham's F12 = 3:1 supplemented with 10% FBS and not supplemented with any ROCK inhibitor) for differentiation induction, followed by medium exchange after 3 days and temperature change to 20°C at day 4 to obtain a nasal mucosal cell sheet (FIG. 6, right), which was then detached from the culture substrate coated with a temperature-responsive polymer.

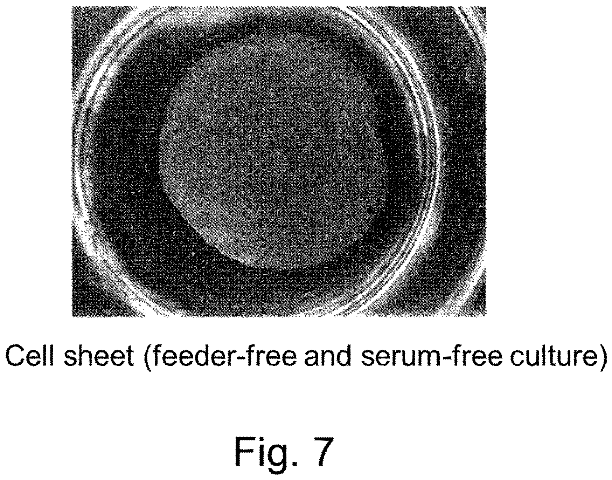

The obtained cell sheets maintained their sheet form (FIG. 7), and were successfully detached while being grasped with tweezers. This demonstrates that the cell sheets have the same strength as that of the mucosal epithelial cell sheets obtained by serum-containing culture using feeder cells in Example 2.

Next, the cell composition of the obtained cell sheets was examined by immunohistochemical staining. The subjects detected were undifferentiated cells expressing p63 that is a marker for epithelial stem/ progenitor cell; and fibroblasts. The cell sheets were prepared using nasal mucosal tissues collected from three human subjects (subject IDs: #8, #9, and #10) as sources, and using a cell culture substrate coated with a temperature-responsive polymer. The detached cell sheets were fixed with paraformaldehyde, then embedded in paraffin, and cut thinly in the vertical direction such that the cross section of the cultured cell sheet could be observed to prepare tissue sections. Undifferentiated cells expressing p63 that is a marker for epithelial stem/ progenitor cell, and fibroblasts were detected.

Undifferentiated cells expressing p63 were detected by reacting the tissue sections with an anti-p63 antibody (Abcam) as a primary antibody, and developing and observing the reaction products using EnVision (DAKO) and DAB (DAKO).

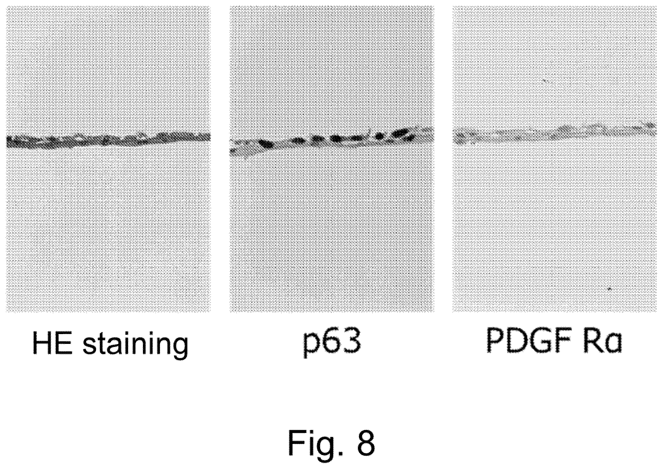

With respect to the expression of p63 based on immunohistochemical staining, the content of undifferentiated cells in the cell sheets prepared using cell culture substrates coated with a temperature-responsive polymer (FIG. 8) was in the range of 88 to 89%.

[Table 3]

**Table 3**

| Subject ID | p63 positive rate (%) | SD |
|---|---|---|
| #8 | 88.48 | 8.84 |
| #9 | 88.08 | 9.99 |
| #10 | 88.12 | 8.54 |
| Overall | 88.23 | 9.14 |

On the other hand, the percentage of fibroblasts with respect to the obtained cell sheets was determined by immunohistochemical staining for PDGFRα (FIG. 8) to find that the percentage of fibroblasts in the cell culture substrates coated with a temperature-responsive polymer was in the range of 0 to 2.9%.

Therefore, nasal mucosal cell sheets prepared by a culture method using a serum-free medium and a method without using feeder cells have the same characteristics as those of cell sheets obtained via serum-containing culture using feeder cells.

### Example 6: Transplantation of cultured cell sheet in rabbit

This Example aimed to transplant a nasal mucosa-like cell sheet prepared according to the present invention to a rabbit whose mucosa in the middle ear cavity was previously removed, and evaluate the effect of the bone tissue-regenerating cell sheet of the present invention.

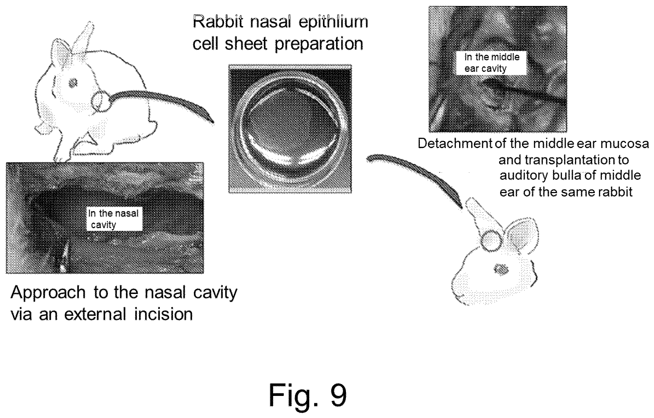

The working process for this is shown in FIG. 9. The cell sheet prepared in Example 3 was used as a cell culture sheet for transplantation. The cell sheet was detached from the cell culture substrate coated with a temperature-responsive polymer, and then the cell sheet wrapped around a blunt-tipped metal needle using forceps was transplanted to the exposed bone surface on the middle ear cavity of a rabbit whose mucosa in the middle ear cavity was previously removed.

As a result, the middle ear cavity was not narrowed and no bone augmentation was observed in the group in which the bone tissue-regenerating cell sheet of the present invention was transplanted, while the middle ear cavity was narrowed and bone augmentation was evident in the group in which the mucosa in the middle ear cavity was removed and nothing was transplanted. The results were also observed in coronal section CT, or in the cases where tissues in the middle ear cavity were collected, decalcified, sectioned, and stained. In addition, analysis of the decalcified sections of the transplantation site demonstrated that engrafted nasal mucosa-like cell sheets remained.

In order to evaluate the function of the middle ear cavity to which the bone tissue-regenerating cell sheet of the present invention had been transplanted, the middle ear cavity of a rabbit was closed by plugging the eustachian tube area. The results showed that the group with transplantation of the mucosal epithelial cell sheet of the present invention was superior to the group in which the middle ear mucosa was removed, and the values were nearly normal. This was considered to be because the air pressure inside and outside the eardrum is maintained at approximately the same level as normal tissues, allowing for maintenance of the optimal function to transmit sounds to the inner ear. Therefore, it is confirmed that the bone tissue-regenerating cell sheet of the present invention can substitute for middle ear mucosae.

### Example 7: Transplantation of cultured cell sheet to human

This Example aimed to transplant a nasal mucosa-like cell sheet prepared according to the present invention to a human patient after surgery for middle ear cholesteatoma or adhesive otitis media, and evaluate the effect of the bone tissue-regenerating cell sheet of the present invention.

After obtaining sufficient oral and written informed consent from the subject human patients, their blood was first collected. This patient's blood was used to confirm the absence of any infection and to prepare autoserum to be added to the culture medium.

Next, human nasal mucosal tissues of approximately 10 × 10 mm in size were endoscopically collected at an outpatient clinic. Human nasal mucosal epithelial cell sheets were then prepared according to the methods described in Examples 1 and 3, using an autoserum-containing KCM (Keratinocyte Culture Medium) medium, in accordance with Master Batch Record prepared in advance, at the cell processing facility (CPF) in the Jikei University School of Medicine.

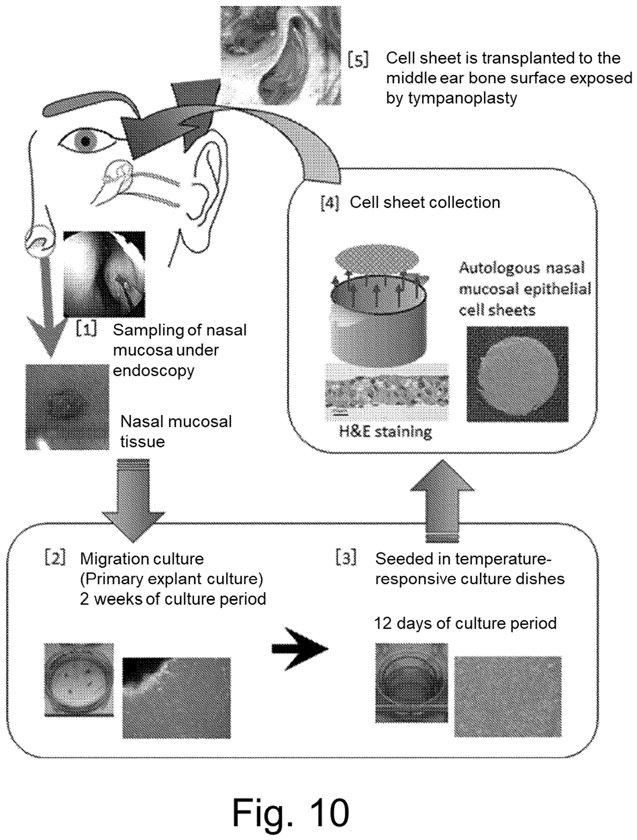

Specifically, collected nasal mucosal epithelia were cultured for two weeks for explant culture according to the method described in Example 1, and then the proliferated epithelial cells were seeded onto a cell culture substrate coated with a temperature-responsive polymer according to the method described in Example 3, followed by passage culture for 12 days, to prepare cell sheets. After confirming no infection during culture, the cell purity, cell survival, and other tests were performed. All the prepared autologous cultured epithelial cell sheets met the established quality standards. Various quality inspection results were checked until the day before transplantation, and cell sheets that met the standards for the specifications were transplanted onto an exposed bone surface where the mucosawas lost during tympanoplasty after removal of cholesteatoma (FIG. 10).

To date, tympanoplasty combined with transplantation of autologous nasal mucosal epithelial cell sheets has been performed in four patients with middle ear cholesteatoma and one patient with adhesive otitis media. During routine tympanoplasty, prepared autologous cultured epithelial cell sheets were transplanted onto the bone surface where the middle ear mucosa had been lost and exposed in order to promote postoperative regeneration of the middle ear mucosa and prevent readhesion of the tympanic membrane. The cell sheets were successfully transplanted onto the bone surface of the narrow middle ear cavity by using a transplantation device.

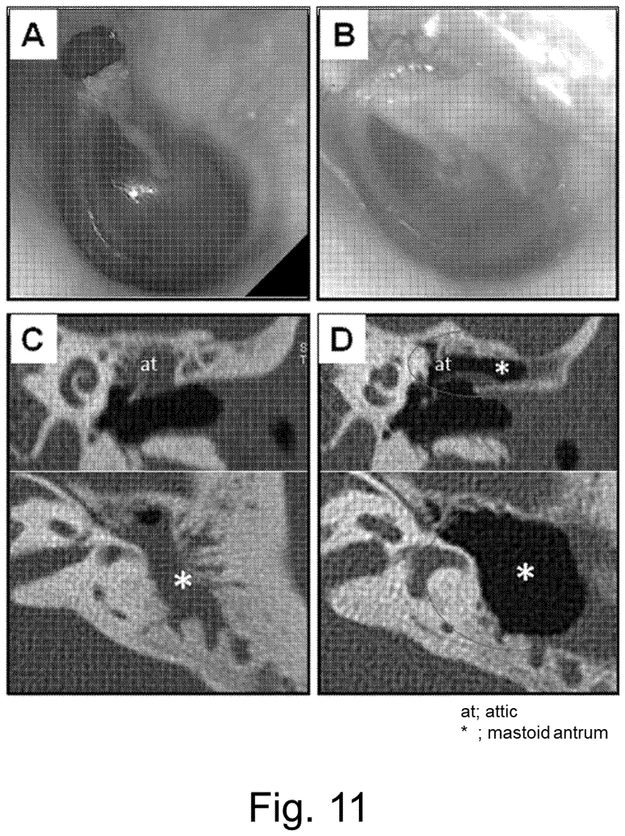

In all cases, the post-transplantation courses were very good, resulting in improved hearing. In addition, postoperative CT showed pneumatization of the middle ear cavity consistent with the transplantation site of the cell sheet, with no evidence of recurrence of cholesteatoma or readhesion of the tympanic membrane (FIG. 11). The longest observation period has been three years or longer, but no adverse events caused by cell sheet transplantation have been observed.

### Industrial Applicability

The cultured cell sheet made from cells collected from a nasal mucosal tissue disclosed in the present invention can be efficiently engrafted onto the surface of middle ear bones, promote the regeneration of mucosal tissues covering bone tissues, and prevent fibrosis, granulation tissue formation, defective epithelial formation, and the like in the middle ear cavity. The cultured cell sheet made from cells collected from a nasal mucosal tissue is also used as a useful mucosa in cases of loss of mucosa in the nasal cavity and oral cavity. Furthermore, a cultured cell sheet made from cells collected from a nasal mucosal tissue of the present invention is also useful for preventing inflammation of bone tissues in other areas of the body other than the middle ear.

## Claims

1. A cultured cell sheet, wherein the cultured cell sheet is made from cells collected from a nasal mucosal tissue as a source, and comprises 50 to 90% of undifferentiated cells relative to the total number of cells.

2. The cultured cell sheet according to claim 1, wherein the undifferentiated cells are positive for p63.

3. The cultured cell sheet according to claim 1 or 2, wherein the undifferentiated cells are nasal mucosal epithelial stem cells or nasal mucosal epithelial progenitor cells.

4. The cultured cell sheet according to claim 1 or 2, wherein the undifferentiated cells do not have cilia.

5. The cultured cell sheet according to claim 1 or 2, wherein the cell sheet is a mixture comprising nasal mucosal epithelial cells and nasal mucosal epithelial stem cells or nasal mucosal epithelial progenitor cells, along with any one or two or more cells of other epithelial cell or other epithelial stem cell, a mesenchymal stem cell, a fibroblast, a vascular endothelial cell, an endothelial progenitor cell, and an adipose-derived stem cell.

6. The cultured cell sheet according to claim 5, wherein the percentage of the fibroblasts contained in the cell sheet is 0 to 12%.

7. The cultured cell sheet according to claim 1 or 2, wherein the nasal mucosal tissue from which the cells of the source are derived is derived from an inferior turbinate mucosa, a middle turbinate mucosa, a superior turbinate mucosa, a nasal mucosa, or a paranasal sinus tissue.

8. The cultured cell sheet according to claim 1 or 2, wherein the nasal mucosal tissue is derived from either human, rabbit, dog, cat, pig, monkey, chimpanzee, rat, or mouse.

9. The cultured cell sheet according to claim 1 or 2, intended for the treatment of chronic otitis media, adhesive otitis media, middle ear cholesteatoma, chronic sinusitis, paranasal sinus neoplasm, external auditory canal tumor, external auditory canal atresia, chronic tonsillitis, pharyngeal cancer, laryngeal cancer, oral cancer, tongue cancer, endotracheal granulation, endotracheal cicatrization, tracheal stenosis, tracheal injury, esophageal cancer, vocal cord lesion, inflammatory bowel disease, anal ulcer, anal fissure, hemorrhoid, or anal fistula.

10. A method of producing a cultured cell sheet, wherein the cultured cell sheet is made from cells collected from a nasal mucosal tissue as a source and comprises 50 to 90% of undifferentiated cells relative to the total number of cells, and the method comprises the following steps of:
(1) preparing mucosal epithelial cells as the source from the nasal mucosal tissue;
(2) culturing the prepared mucosal epithelial cells to increase the number of the mucosal epithelial cells as the source;
(3) seeding the obtained cells onto a cell culture substrate and culturing the cells to prepare a cultured cell sheet in which the cells cover the culture substrate surface, on the culture substrate; and
(4) detaching and collecting the cultured cell sheet prepared on the culture substrate while maintaining the sheet shape.

11. The method according to claim 10, wherein the mucosal epithelial cells as the source are prepared by a following step in the step (1):
preparing the mucosal epithelial cells as the source by obtaining cells by an explant culture method in which the nasal mucosal tissue collected from a subject is left to stand directly on a cell culture substrate without enzyme treatment, and cultured in serum-containing medium or serum-free medium to allow the cells in the tissue to migrate into the medium.

12. The method of producing the cultured cell sheet according to claim 10 or 11, wherein the cell culture substrate used in the steps (1) and (2) is a dish type culture substrate, a flask type culture substrate, or a multiwell type culture substrate.

13. The method according to claim 10 or 11, wherein the mucosal epithelial cells as the source are prepared by following steps in the steps (3) and (4):
(3) culturing the prepared mucosal epithelial cells on a cell culture substrate coated on its surface with a temperature-responsive polymer having varying hydration force in the temperature range of 0 to 80°C, in a temperature range in which the temperature-responsive polymer has weak hydration force to prepare a cultured cell sheet on the cell culture substrate; and
(4) changing the temperature of the culture medium into a temperature at which the temperature-responsive polymer has strong hydration force to detach the cultured cell sheet from the cell culture substrate and obtain the cultured cell sheet while maintaining the sheet shape.

14. The method of producing the cultured cell sheet according to claim 10 or 13, wherein the cell culture substrate used in the step (3) is coated on the surface with one or a mixture of two or more of type I collagen, laminin, fibronectin, and Matrigel.

15. The method of producing the cultured cell sheet according to claim 10 or 13, wherein the temperature-responsive polymer used in the steps (3) and (4) is poly-(N-isopropylacrylamide).

16. The method of producing the cultured cell sheet according to claim 10 or 13, wherein the detaching process in the step (4) is adhering a carrier closely on the cultured cell and detaching the cultured cell together with the carrier.

17. The method of producing the cultured cell sheet according to claim 10 or 13, further comprising laminating the cell sheet detached in the step (4) on another cell sheet detached in the step (4), or repeating the operation to laminate cell sheets.

18. The method of producing the cultured cell sheet according to claim 10 or 13, wherein the number of the mucosal epithelial cells seeded at the start of the culturing in the step (3) is 3 × 10⁴ to 2 × 10⁵ cells/cm².

19. The method of producing the cultured cell sheet according to claim 10 or 13, wherein feeder cells are used in the culturing in the step(s) (2) and/or (3).

20. The method of producing the cultured cell sheet according to claim 10 or 13, wherein a serum-free medium is used in the culturing in the step (3).
